# EUROPEAN PATENT SPECIFICATION

(11) **EP 1 158 001 B1**
(45) Date of publication and mention of the grant of the patent: **14.11.2007**
(21) Application number: 01112004.5
(22) Date of filing: 23.05.2001
(51) Int. Cl.: C07K 14/47, C12Q 1/68

(54) **A nucleic acid which is upregulated in human tumor cells, a protein encoded thereby and a process for tumor diagnosis**
Eine Nukleinsäure, die in menschlichen Tumorzellen aufreguliert wird, ein von dieser kodiertes Protein und ein Verfahren zur Tumordiagnose
Un acide nucléique produit excessivement dans des cellules tumorales humaines, une protéine codée par ledit acide nucléique et un procédé de diagnose de tumeurs

(30) Priority: 26.05.2000 EP 00110953; 15.07.2000 EP 00115369
(43) Date of publication of application: 28.11.2001
(73) Proprietor: F. HOFFMANN-LA ROCHE AG, 4070 Basel (CH)
(72) Inventor: Kaul, Sepp, 69168 Wiesloch (DE); Preiherr, Josef, 82362 Weilheim (DE); Weidle, Ulrich, 80336 München (DE)
(74) Representative: Schreiner, Siegfried

(56) References cited:
- WO-A-00/08210
- WO-A-01/55314
- WO-A-98/59049
- US-A- 6 057 105
- DATABASE STRAND [Online] ID AQ548392, 28 May 1999 (1999-05-28) ZHAO, S. ET AL.: "Use of the BAC end sequences from library RPCI-11 for sequence-ready map building" XP002141756
- PREIHERR, JOSEF; HILDEBRANDT, TOBIAS; KLOSTERMANN, STEFAN; EBERHARDT,SABINE; KAUL, SEPP; WEIDLE, ULRICH H.: "Transcriptional profiling of human mammary carcinoma cell lines reveals PKW, a new tumor-specific gene" ANTICANCER RES., vol. 20, no. 4, 2000, pages 2255-2264, XP001021837

## Description

Breast cancer is a major health problem since every eighth woman in Europe and the US succumbs to this disease (Moustafa, A.S., and Nicolson, G.L., Oncol. Res. 9 (1997) 505-525; Nicolson, G.L., Biochem. Soc. Symp. 63 (1998) 231-242). Treatment includes surgery, radiation, chemotherapy and combinations thereof, depending on the stage of the disease (Schwirzke, M., et al., Anticancer Res. 19 (1999) 1801-1814). A pronounced tropism of metastasis to the bones is characteristic for this disease starting with micrometastic lesions in the bone marrow which finally may outgrow to full-blown metastases. Bone metastasis result in bone fractures and spinal cord depression syndrome often followed by severe pain, aberrant calcium homeostasis and finally lead to death of the patients (Coleman, R.E., and Rubens, R.D., Br. J. Cancer 55 (1987) 61-66).

Analysis of genes involved in breast cancer and in cancer in general revealed a dichotomy with one category of genes with deregulated expression due to mutation and the other category of genes exhibiting changes in their regulation. These findings resulted in the grouping of cancer genes into two classes: class I genes are mutated or deleted, class II genes exhibit no alterations at the DNA level (Sager, R., Science 246 (1989) 1406-1412; Sager, R., Proc. Natl. Acad. Sci. USA 94 (1997) 952-955).

### Summary of the Invention

In accordance with the present invention, a protein and the related gene, termed PKW, is provided which is upregulated in tumor cells, preferably in mammary tumor cells, as compared to their non-tumor counterparts. The PKW gene codes preferably for a polypeptide consisting of SEQ ID NO:2 or SEQ ID NO:4.

The present invention provides a nucleic acid which is upregulated in tumor cells, especially in mammary carcinoma cells, and which codes for a polypeptide which induces tumor progression or metastasis, the nucleic acid being selected from the group consisting of:
(a) SEQ ID NO: 1;
(b) a nucleic acid sequence free of sequence naturally flanking the 5' and the 3' ends of the nucleic acid sequence of (a) which hybridizes under stringent conditions with a nucleic acid probe of the complementary sequence of (a);
(c) a nucleic acid sequence which, because of the degeneracy of the genetic code, is not a sequence of (a) or (b), but which codes for a polypeptide having exactly the same amino acid sequence as a polypeptide encoded by a sequence of (a) or (b); and
(d) a nucleic acid sequence which is a fragment of at least 138 nucleotides of any of the sequences of (a), (b) or (c), and encodes a polypeptide consisting of amino acids of SEQ ID NO: 2 or SEQ ID NO: 4,
with the proviso that said nucleic acid sequence is not identical to the complementary sequence of SEQ ID NO:12.

The nucleic acid encodes a polypeptide consisting of amino acids of SEQ ID NO:2 or SEQ ID NO:4 and having preferably a length of at least 138 nucleotides.

The present invention further provides a purified polypeptide having a sequence of amino acids SEQ ID NO:2 or SEQ ID NO:4.

The present invention further provides a process for detecting the presence or absence of at least one specific nucleic acid or mixture of nucleic acids, or distinguishing between two different sequences in a sample, wherein said sample is suspected of containing said sequence or sequences and is preferably derived from a patient who is suffering from cancer or who is suspected of having cancer, which process comprises the following steps:
(a) incubating said sample under stringent hybridization conditions with a nucleic acid probe which is selected from the group consisting of:
   (i) a nucleic acid sequence of SEQ ID NO:1 or a fragment thereof;
   (ii) a nucleic acid sequence which is complementary to any nucleic acid sequence of (i);
   (iii) a nucleic acid sequence which hybridizes under stringent conditions with the sequence of (i); and
   (iv) a nucleic acid sequence which hybridizes under stringent conditions with the sequence of (ii); and
(b) determining whether said hybridization has occurred.

Preferably, said sequence is not identical to SEQ ID NO:12 and/or has a length of at least 138 nucleotides.

Moreover, the present invention provides a process for determining whether or not a test sample of tissue or fluid of a patient contains tumor cells or is derived from tumor cells, wherein the test sample and a second sample originating from non-tumor cells from the same individual or a different individual of the same species are used, which process comprises the following steps:
(a) incubating each respective sample under stringent hybridization conditions with a nucleic acid probe which is selected from the group consisting of:
   (i) a nucleic acid sequence of SEQ ID NO: 1, or a fragment thereof;
   (ii) a nucleic acid sequence which is complementary to any nucleic acid sequence of (i);
   (iii) a nucleic acid sequence which hybridizes under stringent conditions with the sequence of (i); and
   (iv) a nucleic acid sequence which hybridizes under stringent conditions with the sequence of (ii); and
(b) determining the approximate amount of hybridization of each respective sample with said probe, and
(c) comparing the approximate amount of hybridization of the test sample to an approximate amount of hybridization of said second sample to identify whether or not the test sample contains a greater amount of the specific nucleic acid or mixture of nucleic acids than does said second sample.

The invention further provides a method for the detection of mammary carcinoma as PKW is expressed only in mammary carcinoma cells or in metastatic cells thereof.

### Detailed Description of the Invention

The present invention provides the new gene PKW, proteins coded thereby, and use of the PKW gene for diagnostics and therapeutics, especially in the field of cancer. In particular, the invention involves the identification of said gene PKW in tumor cells, especially in mammary carcinoma cells and tumor-cell-derived material such as DNA and RNA extracts from cells. The invention also relates to the detection of tumor cells.

The invention comprises a nucleic acid (PKW) which has upregulated expression in tumor cells and which is capable of inducing tumor progression and/or metastasis, especially in mammary carcinoma cells. The nucleic acid (PKW) has the sequence SEQ ID NO:1 or is a nucleic acid which, because of the degeneracy of the genetic code, differs from SEQ ID NO:1 and is preferably a nucleic acid which encodes the amino acid sequence of SEQ ID NO:2 or SEQ ID NO:4.

The invention further comprises recombinant polypeptides which are coded by the nucleic acid sequences according to the invention, preferably by the DNA sequence shown in SEQ ID NO:1 or fragments thereof preferably encoding the polypeptides of SEQ ID NO:2 or SEQ ID NO:4.

The PKW polypeptide can occur in natural allelic variations which differ from individual to individual. Such variations of the amino acids are usually amino acid substitutions. However, they may also be deletions, insertions or additions of amino acids to the total sequence. The PKW polypeptide according to the invention - depending, both in respect of the extent and type, on the cell and cell type in which it is expressed- can be in glycosylated or non-glycosylated form. Polypeptides according to the invention can be identified by transfection of PKW-negative non-tumor cells with expression vectors for PKW, establishment of stable transfectants and evaluation of their tumor progression capacity after xenografting into nude mice.

"Polypeptide with PKW activity or PKW" means also a protein with minor amino acid variations but with substantially the same PKW activity. "Substantially the same" means that the activities are of the same biological properties and the polypeptides show at least 90% homology (identity) in amino acid sequence.

The term "nucleic acid molecule or nucleic acid" denotes a polynucleotide molecule which can be, for example, a DNA, RNA, or derivatized active DNA or RNA. DNA and/or RNA molecules are preferred, however.

The term "hybridize under stringent conditions" means that two nucleic acid fragments are capable of hybridization to one another under standard hybridization conditions described in Sambrook et al., Molecular Cloning: A Laboratory Manual (1989) Cold Spring Harbor Laboratory Press, New York, USA. More specifically, "stringent conditions" as used herein refer to hybridization in 6.0 x SSC at 45°C to 55°C, preferably 50°C to 55°C, followed by a wash. This wash can be with 2.0 x SSC at 50°C. Preferably, hybridization is performed using the commercially available Express Hyb^{™} Hybridization Solution of Clontech, which is a non-viscious solution containing no salmon sperm DNA. The stringency of the salt concentration in the wash step can be selected, for example, from about 2.0 x SSC at 50°C, for low stringency, to about 0.2 x SSC at 50°C, for high stringency. In addition, the temperature in the wash step can be increased from low stringency conditions at room temperatures, about 22°C, to high stringency conditions at about 65°C.

The phrase "nucleic acid or polypeptide" as used throughout this application refers to a nucleic acid or polypeptide having a PKW activity which is substantially free of cellular material or culture medium when produced by recombinant DNA techniques, or substantially free of chemical precursors or other chemicals when synthesized chemically. Such a nucleic acid is free of sequences which naturally flank the nucleic acid (i.e. sequences located at the 5' and the 3' ends of the nucleic acid) in the organism from which the nucleic acid is derived.

The polypeptides according to the invention can be produced by recombinant means, or synthetically. Non-glycosylated PKW polypeptide is obtained when it is produced recombinantly in prokaryotes. With the aid of the nucleic acid sequences provided by the invention it is possible to search for the PKW gene or its variants in genomes of any desired cells (e.g. apart from human cells, also in cells of other mammals), to identify these and to isolate the desired gene coding for the PKW proteins. Such processes and suitable hybridization conditions are known to a person skilled in the art and are described, for example, by Sambrook et al., Molecular Cloning: A Laboratory Manual (1989) Cold Spring Harbor Laboratory Press, New York, USA, and Hames, B.D., Higgins, S.G., Nucleic Acid Hybridisation - A Practical Approach (1985) IRL Press, Oxford, England. In this case the standard protocols described in these publications are usually used for the experiments.

With the aid of such nucleic acids coding for a PKW polypeptide, the polypeptide according to the invention can be obtained in a reproducible manner and in large amounts. For expression in prokaryotic or eukaryotic organisms, such as prokaryotic host cells or eukaryotic host cells, the nucleic acid is integrated into suitable expression vectors, according to methods familiar to a person skilled in the art. Such an expression vector preferably contains a regulatable/inducible promoter. These recombinant vectors are then introduced for the expression into suitable host cells such as, e.g., E. coli as a prokaryotic host cell or Saccharomyces cerevisiae, Teratocarcinoma cell line PA-1, sc 9117 (Büttner et al., Mol. Cell. Biol. 11 (1991) 3573-3583), insect cells, CHO or COS cells as eukaryotic host cells and the transformed or transduced host cells are cultured under conditions which allow expression of the heterologous gene. The isolation of the protein can be carried out according to known methods from the host cell or from the culture supernatant of the host cell. Such methods are described for example by Ausubel I., Frederick M., Current Protocols in Mol. Biol. (1992), John Wiley and Sons, New York. Also in vitro reactivation of the protein may be necessary if it is not found in soluble form in the cell culture.

PKW polypeptide can be purified after recombinant production by affinity chromatography using known protein purification techniques, including immunoprecipitation, gel filtration, ion exchange chromatography, chromatofocussing, isoelectric focussing, selective precipitation, electrophoresis, or the like.

The invention further comprises recombinant expression vectors which are suitable for the expression of PKW, recombinant host cells transfected with such expression vectors, as well as a process for the recombinant production of a protein which is encoded by the PKW gene.

The invention further comprises a method for detecting a nucleic acid molecule of gene PKW, comprising incubating a sample (e.g., body fluids such as blood, cell lysates or a reverse transcript of an RNA sample) with the nucleic acid molecule according to the invention and determining hybridization under stringent conditions of said nucleic acid molecule to a target nucleic acid molecule for determination of presence of a nucleic acid molecule which is the PKW gene and therefore a method for the identification of tumor cells and preferably of mammary tumors. Quantitative detection can be performed by PCR techniques, preferably by the use of quantitative RT-PCR using, e.g., the LightCycler^{®} of Roche Diagnostics GmbH, DE.

To determine whether a test sample contains tumor cells, the approximate amount of hybridization of the nucleic acid with the target nucleic acid or nucleic acids is determined. The approximate amount of hybridization need not be determined quantitatively, although a quantitative determination is encompassed by the present invention. Typically, the approximate amount of hybridization is determined qualitatively, for example, by a sight inspection upon detecting hybridization. For example, if a gel is used to resolve labelled nucleic acid which hybridizes to target nucleic acid in the sample, the resulting band can be inspected visually. When performing a hybridization of isolated nucleic acid which is free from tumor cells from an individual of the same species, the same protocol is followed. One can compare the approximate amount of hybridization in the test sample to the approximate amount of hybridization in the sample free from tumor cells, to identify whether or not the test sample contains a greater amount of the target nucleic acid or nucleic acids than does the sample which is free from tumor cells.

In a further method according to the invention no second sample is used. For the detection whether the expression of PKW gene is upregulated, the level of mRNA of PKW is compared with the level of mRNA of a standard gene (housekeeping gene (see, e.g., Shaper, N.L., et al., J. Mammary Gland Biol. Neoplasia 3 (1998) 315-324; Wu, Y.Y., et al., Acta Derm. Venerol. 80 (2000) 2-3) of the cell, preferably by RT-PCR.

For visual inspection in particular, it is recommended that an appreciable difference by visualized to assess that the test sample contains a greater amount of the target nucleic acid or nucleic acids.

As is shown in accordance with the present invention, the PKW nucleic acid is expressed in a greater amount in a tumor sample than in a sample free from tumor cells and/or in a greater amount than a housekeeping gene. A test sample containing tumor cells will have a greater amount of the PKW nucleic acid than does a sample which is free from tumor cells. To identify a test sample as containing upregulated PKW nucleic acid, i.e., wherein the cells are tumor cells or are tumor cells of a mammary carcinoma, it is preferable that the test sample have an approximate amount of PKW nucleic acid which is appreciably greater that the approximate amount in a sample free of tumor cells. For example, a test sample having an upregulated PKW gene may have approximately 15- to approximately 60-fold greater amount of PKW gene than a sample free of tumor cells or an at least 3-fold greater amount of PKW mRNA than mRNA of a housekeeping gene like glycerolaldehyde-3-phosphate dehydrogenase (GPDH) or porphobilinogen deaminase.

On the basis of the nucleic acids provided by the invention it is possible to provide a test which can be used to detect nucleic acids with upregulated expression in human tumor cells. Such a test can be carried out by means of nucleic acid diagnostics. In this case the sample to be examined is contacted with a probe that is selected from the group comprising
a) the nucleic acid sequence shown in SEQ ID NO:1, fragments thereof or a nucleic acid sequence which is complementary to one of these nucleic acid sequences, and
b) nucleic acids which hybridize under stringent conditions with one of the nucleic acids from a), wherein
the nucleic acid probe is incubated with the nucleic acid of the sample and the hybridization is detected optionally by means of a further binding partner for the nucleic acid of the sample and/or the nucleic acid probe. For obtaining a nucleic acid by hybridization in accordance with b), it is preferable to hybridize to a probe selected from the group of nucleic acids defined by the bases 724 to 1235 (small transcript) or bases 1831-2342 (large transcript) of SEQ ID NO:1, a fragment thereof of at least 200 bases or a sequence complementary thereto. Hybridization between the probe used and nucleic acids from the sample indicates the presence of the RNA of such proteins.

Methods of hybridization of a probe and a nucleic acid are known to a person skilled in the art and are described, for example, in WO 89/06698, EP-A 0 200 362, U.S. Patent No. 2,915,082, EP-A 0 063 879, EP-A 0 173 251, EP-A 0 128 018.

In a preferred embodiment of the invention the coding nucleic acid of the sample is amplified before the test, for example by means of the known PCR technique. Usually a derivatized (labeled) nucleic acid probe is used within the framework of nucleic acid diagnostics. This probe is contacted with a denatured DNA, RNA or RT-DNA from the sample which is bound to a carrier and in this process the temperature, ionic strength, pH and other buffer conditions are selected - depending on the length and composition of the nucleic acid probe and the resulting melting temperature of the expected hybrid - such that the labeled DNA or RNA can bind to homologous DNA or RNA (hybridization see also Wahl, G.M., et al., Proc. Natl. Acad. Sci. USA 76 (1979) 3683-3687). Suitable carriers are membranes or carrier materials based on nitrocellulose (e.g., Schleicher and Schüll, BA 85, Amersham Hybond, C.), strengthened or bound nitrocellulose in powder form or nylon membranes derivatized with various functional groups (e.g., nitro groups) (e.g., Schleicher and Schüll, Nytran; NEN, Gene Screen; Amersham Hybond M.; Pall Biodyne).

Hybridizing DNA or RNA is then detected by incubating the carrier with an antibody or antibody fragment after thorough washing and saturation to prevent unspecific binding. The antibody or the antibody fragment is directed towards the substance incorporated during hybridization to the nucleic acid probe. The antibody is in turn labeled. However, it is also possible to use a directly labeled DNA. After incubation with the antibodies it is washed again in order to only detect specifically bound antibody conjugates. The determination is then carried out according to known methods by means of the label on the antibody or the antibody fragment.

The detection of the expression can be carried out for example as:
- in situ hybridization with fixed whole cells, with fixed tissue smears,
- colony hybridization (cells) and plaque hybridization (phages and viruses),
- Southern hybridization (DNA detection),
- Northern hybridization (RNA detection),
- serum analysis (e.g., cell type analysis of cells in the serum by slot-blot analysis),
- after amplification (e.g., PCR technique).

Therefore the invention also includes a method for the detection of carcinoma cells, comprising
a) incubating a sample of a patient suffering from cancer, selected from the group of body fluid, of cells, or of a cell extract or cell culture supernatants of said cells, whereby said sample contains nucleic acids with a nucleic acid probe which is selected from the group consisting of
   (i) the nucleic acid shown in SEQ ID NO:1 or a nucleic acid which is complementary to said sequence, and
   (ii) nucleic acids which hybridize with one of the nucleic acids from (i) and
b) detecting hybridization, preferably by means of a further binding partner of the nucleic acid of the sample and/or the nucleic acid probe or by X-ray radiography.

In addition, the invention comprises a process for determining whether or not a test sample originating from or containing human cells has a tumor progression potential, which process comprises the following steps:
(a) incubating a first compartment of said sample under stringent hybridization conditions with a first nucleic acid probe which is selected from the group consisting of:
   (i) a nucleic acid with a sequence of SEQ ID NO:1 or a fragment thereof;
   (ii) a nucleic acid with a sequence which is complementary to any nucleic acid of (i);
   (iii) a nucleic acid with a sequence which hybridizes under stringent conditions with the nucleic acid of (i); and
   (iv) a nucleic acid with a sequence which hybridizes under stringent conditions with the nucleic acid of (ii); and
(b) incubating a second compartment of said sample under stringent hybridization conditions with a second nucleic acid probe being a housekeeping gene or a fragment thereof;
(c) determining the approximate amount of hybridization of said sample with said first and second probe;
(d) identifying whether or not the test sample contains an at least 3-fold amount of nucleic acid hybridizing with the first probe in comparison to the amount of nucleic acid hybridizing with the second probe.

Preferably, the nucleic acid probe is incubated with the nucleic acid of the sample and the hybridization is detected optionally by means of a further binding partner for the nucleic acid of the sample and/or the nucleic acid probe. As probes, nucleic acids selected from the group consisting of nucleid acids defined by the bases 724 to 1235 (small transcript) or bases 1831-2342 (large transcript) of SEQ ID NO:1, a fragment thereof of at least 200 bases or a sequence complementary thereto are preferred.

The nucleic acids according to the invention are hence valuable markers in the diagnosis and characterization of tumors, especially of mammary tumors.

The invention further comprises a method for producing a protein whose expression is correlated with tumors, by expressing an exogenous DNA in prokaryotic or eukaryotic host cells and isolation of the desired protein, wherein the protein is coded by the nucleic acid molecules according to the invention, preferably by the DNA sequence shown in SEQ ID NO:1.

The protein can be isolated from the cells or the culture supernatant and purified by chromatographic means, preferably by ion exchange chromatography, affinity chromatography and/or reverse phase HPLC.

The invention further comprises a protein according to the invention which is encoded by a nucleic acid molecule according to the invention, preferably having the nucleotide sequence set forth in SEQ ID NO:1.

The present invention relates to the cloning and characterization of the gene PKW, which is especially characterized as a tumor progression gene, and as an upregulated gene indicative for the tumor progression potential of tumor cells, preferably of mammary tumor cells.

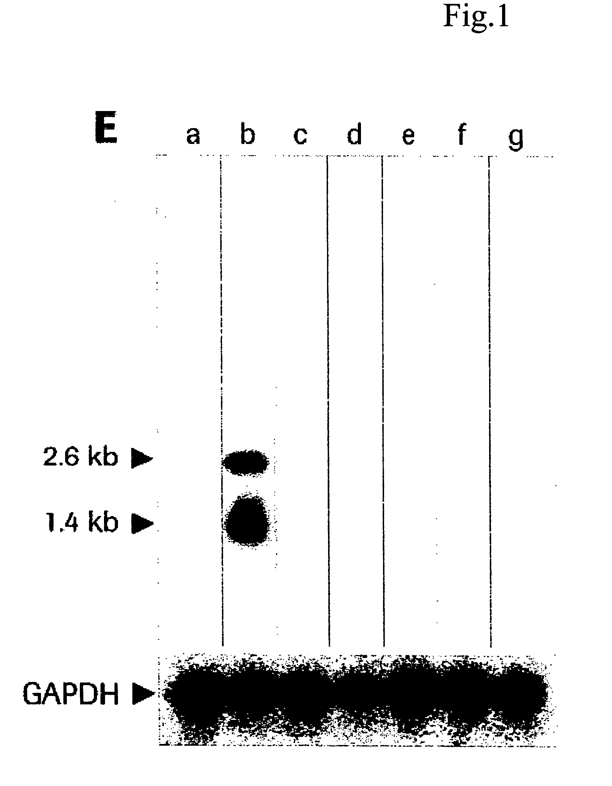

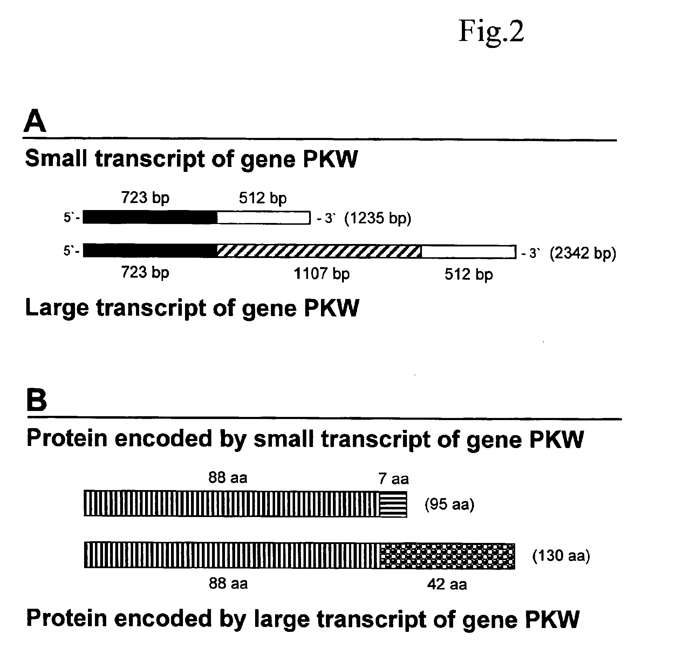

As shown in Fig. 1, gene PKW is expressed only in one of the primary carcinoma cell lines, the one derived from the medullary mammary carcinoma. Topology of small and large transcripts of gene PKW as well as the potential proteins encoded by them are outlined schematically in Fig. 2. Small and large transcript of gene PKW share 723 bp at the 5'end and 512 bp at the 3'end. The large transcript contains an insertion of 1107 bp (Fig. 2A). The small transcript (bp 459-723 and 1831-1850 of SEQ ID NO:1) is due to differential splicing of the large transcript. The small transcript encodes a potential protein of 95 aa, the large transcript exhibits an open reading frame of 130 aa. Both potential proteins share an open reading frame of 88 aa with exception of one different aa at position 43 (nucleic acid position 586 of SEQ ID NO:1), followed by extensions of 7 aa and 42 aa for the smaller and the larger protein in different reading frames. The difference at position 43 may be a PCR artefact or could be caused by polymorphism. The 95 aa protein exhibits an isoelectric point (IEP) of 11.2, the IEP of the 130 aa protein is 10.4. These findings point to a nuclear localization of the proteins encoded by gene PKW. A search revealed partial similarity with a clone derived from a human BAC library (AQ548392) (SEQ ID NO:12). However, the database does not provide any hints as to coding sequences and/or usefulness.

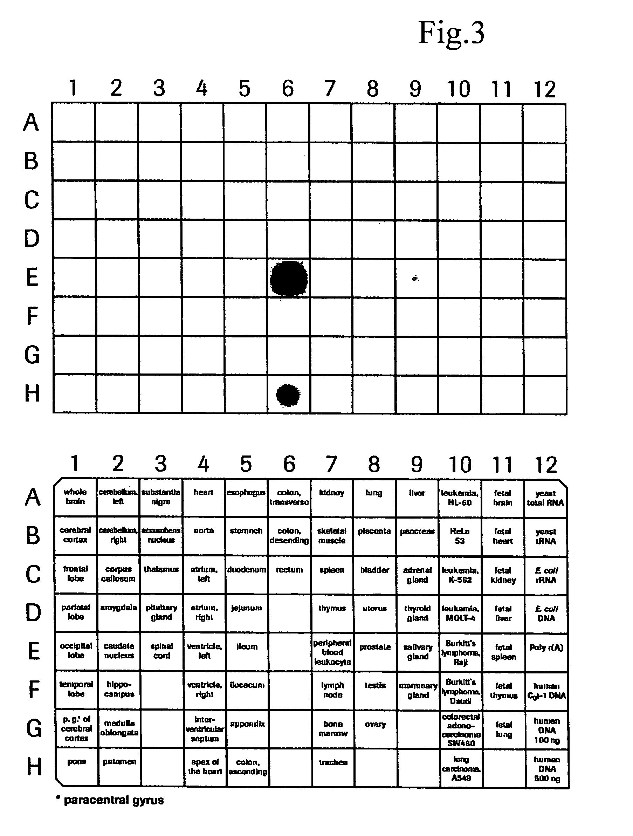

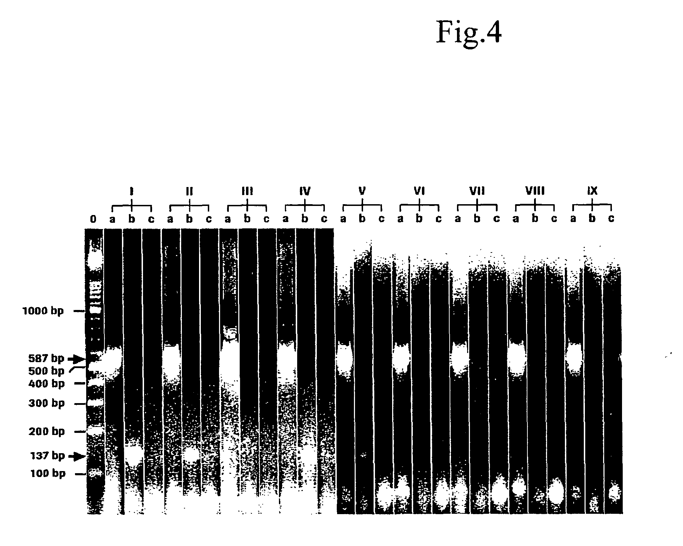

As shown in Fig. 3 transcripts of gene PKW were detected only in salivary gland, not in other adult human tissues and in a small panel of embryonic tissues such as fetal brain, heart, kidney, liver, spleen, thymus and lung. Promyelocytic leukemia cell line HL-60, HeLa cells, chronic myelogenous leukemia cell line K-562, lymphoblastic leukemia cell line MOLT-4, Burkitt lymphoma cell line Raji, colorectal adenocarcinoma cell line SW 480, lung carcinoma cell line A549 and melanoma cell line G361 all scored negative with respect to mRNA for gene PKW (Fig. 3). The probe used for hybridization detects the small as well as the large transcript of gene PKW. A panel of mammary carcinoma cell lines described in (Schwirzke, M., et al., Anticancer Res. 18 (1998) 1409-1421) also tested negative with respect to the mRNA of gene PKW by Northern blotting as well as RT-PCR. These include MDA-435 (Cailleau, R., et al., In Vitro 14 (1978) 911-915) derived subclones 4C4 and 2A5, cell lines MDA-MB231 (Cailleau, R., et al., J. Natl. Cancer Inst. 53 (1974) 661-674), MDA-MB436 (Cailleau, R., et al., In Vitro 14 (1978) 911-915), ZR-75 (Engel, L.W., et al., Cancer Res. 38 (1978) 3352-3364), T47D (Freake, H.C., et al., Biochem. Biophys. Res. Commun. 101 (1981) 1131-1138), Hs578 T (Hackett, A.J., J. Natl. Cancer Inst. 58 (1977) 1795-1806), MCF-7 (Schiemann, S., et al., Anticancer Res. 17 (1997) 13-20; Schiemann, S., et al., Clin. Exp. Metastasis 16 (1998) 129-139), MCF-7_{ADR} (Schiemann, S., et al., Anticancer Res. 17 (1997) 13-20; Lee, J.H., et al., Biochem. Biophys. Res. Commun. 238 (1997) 462-467), LCC-1, LCC-2 and LCC-9 (Brünner, N., et al., Cancer Res. 53 (1993) 283-290; Brünner, N., et al., Cancer Res. 53 (1993) 3229-3232). In summary 11 mammary carcinomas were analyzed for expression of the small transcript of gene PKW by RT-PCR. 4 carcinomas scored positive. Parts of the results are displayed in Fig. 4. Two of the positive carcinomas corresponded to ductal carcinomas and the other two matched with lobular carcinomas.

The following examples, references, sequence listing and figures are provided to aid the understanding of the present invention.
- **SEQ ID NO:1:**: cDNA of PKW gene and amino acid sequence of large PKW splice variant.
- **SEQ ID NO:2:**: Amino acid of large PKW splice variant.
- **SEQ ID NO:3 :**: cDNA and amino acid sequence of small PKW splice variant
- **SEQ ID NO:4:**: Amino acid of small PKW splice variant.
- **SEQ ID NO:5:**: Primer GSP1
- **SEQ ID NO:6:**: Primer GSP2
- **SEQ ID NO:7 :**: Primer AUAP
- **SEQ ID NO:8 :**: Primer RTR-5
- **SEQ ID NO:9 :**: Primer RTF-6
- **SEQ ID NO:10 :**: β-actin reverse primer
- **SEQ ID NO:11 :**: β-actin forward primer
- **SEQ ID NO:12 :**: Fragment of sequence AQ 548392

### Description of the Figures

- **Figure 1**: Northern blot revealing differentially expressed mRNA's in cell lines derived from normal human mammary gland and from breast carcinomas of distinct stages of progression.
RNA was extracted from confluent cell lines, separated on a denaturing 1% agarose-formaldehyde gel, transferred to a positively charged nylon membrane and hybridized to an [α-³²P]-labeled probe corresponding to the appropriate subcloned fragment as revealed by the Differential Display Technique.
Lane a: HMEC, normal Human Mammary Epithelial Cells; lane b: cell line AR derived from medullary mammary carcinoma; lane c: cell line WA derived from invasive ductal mammary carcinoma; lanes d, e and f: cell lines 1590, HG15 and KM22, derived from mammary carcinoma bone marrow micrometastases; lane g: metastatic mammary carcinoma cell line KS, derived from malignant ascites fluid.
- **Figure 2**: Schematic outline of transcripts of gene PKW as well as potential proteins encoded by these transcripts. Corresponding regions and domains are highlighted by conserved symbols.
- **Figure 3**: Multiple Tissue Array
Normalized poly A+ RNA from different tissues and cell lines were hybridized to a 32P-labeled probe derived from gene PKW. E6 corresponds to 1 µg and H6 to 0.1 µg poly A⁺ RNA from cell line AR. The code is revealed below.
- **Figure 4**: Detection of transcripts of gene PKW in mammary carcinomas by RT-PCR.
RNA was extracted from mammary carcinomas and analyzed for transcripts corresponding specifically to the small transcript of gene PKW as described in Example 9.
Lane 0: DNA Molecular Weight Marker XIV (Roche Diagnostics GmbH, DE);
I: cell line AR; II-IX: different mammary carcinomas samples;
lane a: β actin control (2.5 µg RNA + specific primers for β actin); lane b: (2.5 µg RNA + specific primers for gene PKW); lane c: negative controls without RT: 2.5 µg RNA + specific primers for gene PKW.
An aliquot (15 µl) of the PCR products was analyzed on a 1.5% agarose gel. The bands specifically corresponding to mRNAs for gene PKW (137 bp) and β-actin (587 bp) are depicted by arrows.

### Example 1

### Cell lines and cell culture

Human mammary epithelial cells (HMEC) were obtained from Bio Whittaker, Heidelberg, Germany. Primary mammary carcinoma cell lines AR and WA were obtained by fragmenting the primary tumor with scissors, treatment with collagenase (0.2 mg/ml in 5% FCS) and finally the cellular fraction was isolated by Ficoll gradient technique. Tumor cells were selected with a monoclonal antibody directed against MUC-1 coated to Dynabeads^{®} (Dynal, Norway). MUC-1 antibodies are described in WO 99/40881. 5 x 10⁷ beads were mixed with 10⁷ cells, incubated for 1h at 4°C on a roller device, the bead fraction was collected on a magnet, washed twice with DMEM and finally the cells were propagated in 75cm² culture flasks in DMEM supplemented with 10%FCS. Two clones were identified after 4 weeks for both cell lines referred to as AR and WA. Cell line AR is derived from an invasive medullary mammary carcinoma, cell line WA is derived from an invasive ductal carcinoma. Cell lines 1590, HG15 and KM22 are derived from bone marrow micrometastases of mammary carcinoma patients. Cellular fraction was isolated on a Ficoll gradient, erythrocytes were lysed and the cells were suspended in DMEM + 10%FCS and tumor cells were isolated on Dynabeads^{®} coupled with MUC-1 antibody as described above. The bead fraction was cultivated in DMEM + 10%FCS, 10 µg/ml insulin and 10 µg/ml transferrin. Outgrowth of tumor cell lines was observed after 8 weeks. Clones were isolated by treatment with EDTA and propagated under standard conditions as described above. Cell line KS was isolated from malignant ascites fluid of a mammary carcinoma patient. 2000 ml of ascites were collected and the cellular fraction was isolated on a Ficoll gradient. 2 x 10⁷ cells were seeded into 750 cm² culture flasks. Tumor cell clusters were obtained from the culture supernatants in order to separate them from adherently growing fibroblasts and mesothelial cells (passages 1-4). Passages 5-10 resulted in cultures growing partly as a monolayer and in suspension. Finally cells were propagated as a monolayer in DMEM + 10% FCS.

### Example 2

### mRNA Differential Display PCR

Differential display reverse transcriptase polymerase chain reaction (DD-RT-PCR) was performed following the method described by Liang and Pardee using the RNAimage^{™} kits (GenHunter Corp. Brookline, MA) according to the manufacturer's protocol.

Total RNA was isolated from frozen cell pellets of all cell lines listed above making use of the RNeasy Midi^{®} Kit (Qiagen, www.qiagen.de). Chromosomal DNA was removed from RNA samples by digestion at 37°C for 30 min with RNAse-free DNAse I using the MessageClean Kit^{®} (GenHunter Corp. Brookline, MA).

RNA was used as a template for first strand cDNA synthesis in the presence of 3 different one-base anchored oligo-dT primers (H-T₁₁M, where M may be G, A or C).

For a 20 µl reaction, 1 µl of DEPC-treated H₂O, 4 µl of 5x reverse transcriptase buffer [125 mM Tris-Cl, pH 8.3, 188 mM KCl, 7.5 mM MgCl₂, 25 mM dithiothreitol (DDT)], 10 µl of dNTP mix [250 µM each], 2 µl of H-T₁₁M primer [2 µM], and 2 µl of DNA-free total RNA sample [0.1 µg/µl] were mixed. The solution was heated to 65°C for 5 min and cooled to 37°C for 10 min, and 1 µl [100 units] of Moloney murine leukemia virus (MMLV) reverse transcriptase was added. After incubation at 37°C for 1h, the reaction was terminated by incubation at 75°C for 5 min. The following PCR procedure was performed in a 20 µl reaction, containing 2 µl of reverse transcription reaction mixture, 9.2 µl of DEPC-treated H₂O, 2 µl pf 10x PCR buffer [100 mM Tris-Cl, pH 8.4, 500 mM KCl, 15 mM MgCl₂, 0.01% gelatine], 1.6 µl of dNTP mix [25 µM each], 2 µl of the respective H-T₁₁M primer [2 µM], 2 µl of an arbitrary 13-mer primer, [2 µM], 1 µl of α-[³⁵S]dATP [>1000 Ci/mmole] and 0.2 µl of AmpliTag [10 units/µl] DNA polymerase (Perkin Elmer, Norwalk, CT). PCR included a total of 40 cycles at 94°C for 30s, 40°C for 2 min, 72°C for 30 s, and finally 5 min at 72°C. After adding 2 µl loading buffer to 3.5 µl of each sample, the PCR products of all cell lines were heated at 80°C for 2 min and loaded in parallel on a denaturing 6% polyacrylamide sequencing gel for electrophoresis. The dried gel was exposed to BioMax^{™} MR film (Kodak) at room temperature for 24 to 48 h and the autoradiogram was analyzed for the differentially expressed genes. Bands corresponding to cDNA's of interest reproducibly displayed in two independent DD-RT-PCR reactions were excised from the dried gel, and the cDNA was eluted from the gel by soaking the gel slice in 100 µl of dH₂O of 10 min and boiled for 15 min. After addition of 10 µl of 3M NaOAc and 5 µl glycogen [10mg/ml] as carrier the cDNA-fragments were recovered by precipitation with 450 µl of ethanol and redissolved in 10 µl dH₂O. 4 µl eluted cDNA was reamplified in a second PCR using the same primer set and conditions except the dNTP concentrations of 20 µM each and no radioistope. As a control, gel slices were excised from lanes without visible bands on a level with the detected cDNA fragments of interest and treated as described above. The amplified PCR products obtained were analyzed on a 3% NuSieve^{®} GTG (FMC BioProducts, Rockland), agarose gel, then purified using the QIAquick^{™} Gel Extraction kit (Qiagen, DE) and used as probes for Northern analysis.

### Example 3

### DNA sequencing of DD-RT-PCR fragments

All PCR fragments of interest were sequenced directly after extraction and purification from agarose gels. The nucleotide sequences data were analyzed for homologies with known genes or EST's in the current DNA data bases.

### Example 4

### Northern blot analysis

Poly A⁺-RNA was isolated from total RNA. 1 µg of polyA⁺-RNA from HMEC, AR, WA, 1590, KM22, HG15 and KS cells were loaded side by side on a denaturing 1% agarose formaldehyde gel and then size-separated by electrophoresis. Blotting to positively charged nylon membrane was done by capillary downward transfer. After UV-crosslinking (Stratagene UV Stratalinker^{™} 2400, www.stratagene.com) blots were hybridized. For that the DD-RT-PCR products were labeled with α-[³²P]dATP up to a specific activity of 2x 10⁹ cpm/µg. Prehybridization (30 min) and hybridization (over-night) with radioactive probes were performed in ExpressHyb^{™} hybridization solution (Clontech, www.clontech.com) at 68°C according to the manufacturer's recommendation. Membranes were washed in solution 1 (2x SSC, 0.05% SDS) at room temperature for 30-40 min with continuous agitation and several replacements of the wash solution 1 followed by a washing step with solution 2 (0.1 x SSC, 0.1% SDS) at 50°C for 40 min with one change of fresh solution. The membranes were then exposed to Cronex^{™}, Medical X-Ray Films (Sterling Diagnostic Imaging Inc., USA) at -80°C for 3 to 72 h. Equal loading and transfer of mRNA to the membrane was assessed by rehybridizing the blots with α-[³²P]dATP-labeled glyceraldehyde-3-phosphate dehydrogenase (GAPDH).

### Example 5

### Cloning of DD-RT-PCR fragments

Northern analysis was first performed using hybridization probes generated directly by PCR reamplification. Those amplified PCR fragments corresponding to differentially expressed mRNAs on a Northern blot were subcloned Subcloned fragments were isolated and stored for further experiments to verify differential expression.

### Example 6

### 5' RACE PCR

This method was applied to isolate the cDNA's of gene PKW. To identify the 5'-sequences of both transcripts a 5'RACE (Rapid Amplification of cDNA Ends) PCR was performed following the manual as described in the 5' RACE System for Rapid Amplification of cDNA Ends Kit, Version 2.0 (Gibco BRL, Life Technologies). First strand cDNA was synthesized from total RNA (without digestion with DNase I) using the gene-specific primer GSP1 (5'TTATCTTTATTCATTTTGG-3', SEQ ID NO:5) and SuperScript^{™} II, an RNAse H⁻ derivative of the Moloney Murine Leukemia Virus Reverse Transcriptase (M-MVL RT). After cDNA synthesis, the solution was purified from unincorporated dNTPs and GSP1. TdT (Terminal deoxynucleotidyl transferase) was used to add homopolymeric tails to the 3'ends of the cDNA. Tailed cDNA then was amplified by PCR using a nested, gene-specific primer GSP2(5'TGCGGGACTCGTCGTAAGTATGC-3', SEQ ID NO:6),which anneals 3' to GSP1, and the deoxyinosine-containing abriged universal amplification primer AUAP (5'GGCCACGCGTCGACTAGTAC-3', SEQ ID NO:7). After several reactions with varying parameters also the longer cDNA (corresponding to the 2.6 kb transcript) could be detected in addition to the shorter one, which appears in any reaction. The enriched and purified cDNA's were cloned and sequenced.

### Example 7

### Human Multiple Tissue Expression Array (MTE^{™})

This array (Clontech, Palo Alto, CA) contains normalized loadings of poly A⁺-RNA from 76 different human tissues as well as control RNAs and DNAs as revealed in Fig 5. The blot was hybridized with an α-[³²P]dATP PKW cDNA according to the instructions of the manufacturer and exposed to X-ray film at -70°C.

### Example 8

### Isolation of RNA from Breast Tumor Tissues

Total RNA was isolated from frozen tumor samples. The frozen tissues were covered with the suggested amount of lysis buffer and immediately disrupted and homogenized by means of a homogenizer for 45-60 sec and 20000 U/min. The homogenized lysate was further processed as described in the manufacturer's protocol.

### Example 9

### Reverse Transcription Polymerase Chain Reaction (RT-PCR)

In order to eliminate genomic DNA contamination the total RNA samples were treated with RNAse-free DNAseI at 37°C for 30 min. First strand synthesis was performed following the protocol of the first strand cDNA Synthesis Kit for RT-PCR (Roche Diagnostics GmbH, DE) by using the specific reverse primer RTR-5 (5'CCATTCATTCATTTTCAAG3', SEQ ID NO:8). The reverse transcription reaction was performed at 25°C for 10 min and then at 55°C for 60 min. After incubation, the AMV Reverse Transcriptase was denatured at 99°C for 5 min. For each sample a negative control reaction without AMV Reverse Transcriptase was performed.

The resulting single-stranded cDNA was amplified by PCR (High Fidelty PCR Master, Roche Diagnostics GmbH, DE) utilizing a second specific forward primer RTF-6 (5'AAAACGCATGGCTTGTC3', SEQ ID NO:9). The amplification was performed with an initial denaturation step at 94°C for 2 min, 10 cycles of 15 s denaturation at 94°C, 30 s annealing at 57°C and 1 min elongation at 72°C, followed by cycles under same conditions. Equal loading and integrity of mRNA was assessed by a control RT-PCR with β-actin primers (reverse primer 5'AGGGTACATGGTGGTGCCGCCAGAC3' SEQ ID NO:10 forward primer 5'CCAAGGCCAACCGCGAGAAGATGAC3' SEQ ID NO:11).

### List of References

Ausubel I., Frederick M., Current Protocols in Mol. Biol. (1992), John Wiley and Sons, New York
Brünner, N., et al., Cancer Res. 53 (1993) 283-290
Brünner, N., et al., Cancer Res. 53 (1993) 3229-3232
Büttner et al., Mol. Cell. Biol. 11 (1991) 3573-3583
Cailleau, R., et al., In Vitro 14 (1978) 911-915
Cailleau, R., et al., J. Natl. Cancer Inst. 53 (1974) 661-674
Coleman, R.E., and Rubens, R.D., Br. J. Cancer 55 (1987) 61-66
Engel, L.W., et al., Cancer Res. 38 (1978) 3352-3364
EP-A 0 063 879
EP-A 0 128 018
EP-A 0 173 251
EP-A 0 200 362
Freake, H.C., et al., Biochem. Biophys. Res. Commun. 101 (1981) 1131-1138
Hackett, A.J., J. Natl. Cancer Inst. 58 (1977) 1795-1806
Hames, B.D., Higgins, S.G., Nucleic Acid Hybridisation - A Practical Approach (1985) IRL Press, Oxford, England
Lee, J.H., et al., Biochem. Biophys. Res. Commun. 238 (1997) 462-467
Liang, P., and Pardee, A.B., Science 257 (1992) 967-971
Liang, P., et al., Cancer Res. 52 (1992) 6966-6968
Liang, P., et al., Nucleic Acids Res. 21 (1993) 3269-3275
Moustafa, A.S., and Nicolson, G.L., Oncol. Res. 9 (1997) 505-525
Nicolson, G.L., Biochem. Soc. Symp. 63 (1998) 231-242
Sager, R., Proc. Natl. Acad. Sci. USA 94 (1997) 952-955
Sager, R., Science 246 (1989) 1406-1412
Sambrook et al., Molecular Cloning: A Laboratory Manual (1989) Cold Spring Harbor Laboratory Press, New York, USA
Schiemann, S., et al., Anticancer Res. 17 (1997) 13-20
Schiemann, S., et al., Clin. Exp. Metastasis 16 (1998) 129-139
Schwirzke, M., et al., Anticancer Res. 18 (1998) 1409-1421
Schwirzke, M., et al., Anticancer Res. 19 (1999) 1801-1814
Shaper, N.L., et al., J. Mammary Gland Biol. Neoplasia 3 (1998) 315-324
U.S. Patent No. 2,915,082
Wahl, G.M., et al., Proc. Natl. Acad. Sci. USA 76 (1979) 3683-3687
WO 89/06698
Wu, Y.Y., et al., Acta Derm. Venerol. 80 (2000) 2-3

## Claims

1. A nucleic acid which is upregulated in mammary tumor cells, said nucleic acid being selected from the group consisting of:
(a) SEQ ID NO: 1;
(b) a nucleic acid sequence free of sequences naturally flanking the 5' and the 3' ends of the nucleic acid sequence of (a) which hybridizes under stringent conditions with a nucleic acid probe of the complementary sequence of (a);
(c) a nucleic acid sequence which, because of the degeneracy of the genetic code, is not a sequence of (a) or (b), but which codes for a polypeptide having exactly the same amino acid sequence as a polypeptide encoded by a sequence of (a) or (b); and
(d) a nucleic acid sequence which is a fragment of at least 138 nucleotides of any of the sequences of (a), (b) or (c) and encodes a polypeptide consisting of amino acids of SEQ ID NO:2 or SEQ ID NO:4,
with the proviso that said nucleic acid sequence is not identical to the complementary sequence of SEQ ID NO:12.

2. A nucleic acid according to claim 1, having a sequence which codes for a polypeptide of amino acids of SEQ ID NO:2 or SEQ ID NO:4.

3. The nucleic acid according to claim 1, wherein the nucleic acid has a sequence of nucleotides 459 to 848 of SEQ ID NO:1 or of nucleotides 1 to 285 of SEQ ID NO:3.

4. An expression vector comprising a nucleic acid of claims 1 to 3.

5. A host cell transformed by a nucleic acid of claims 1 to 3.

6. A polypeptide which induces tumor progression, wherein said polypeptide is encoded by a nucleic acid selected from the group consisting of:
(a) SEQ ID NO: 1;
(b) a nucleic acid sequence which hybridizes under stringent conditions with a nucleic acid probe of the complementary sequence of (a) ; and
(c) a nucleic acid which is a fragment of any of at least 138 nucleotides of the sequences of (a) or (b) and encodes a polypeptide consisting of amino acids of SEQ ID NO:2 or SEQ ID NO:4.

7. The polypeptide according to claim 6, wherein the nucleic acid sequence of (b) hybridizes in 6.0 X SSC at 45°C to 55°C, followed by a wash with from about 2.0 X SSC to about 0.2 X SSC at a temperature from about 50°C to about 65°C.

8. A process for detecting the presence or absence of at least one specific nucleic acid or mixture of nucleic acids, or distinguishing between two different nucleic acids in a sample, wherein said sample is suspected of containing said nucleic acid or acids, which process comprises the following steps in order:
(a) incubating said sample under stringent hybridization conditions with a nucleic acid probe which is selected from the group consisting of:
(i) a nucleic acid with a sequence taken from the group consisting of SEQ ID NO: 1 or a fragment thereof;
(ii) a nucleic acid with a sequence which is complementary to any nucleic acid of (i);
(iii) a nucleic acid with a sequence which hybridizes under stringent conditions with the nucleic acidof (i); and
(iv) a nucleic acid with a sequence which hybridizes under stringent conditions with the nucleic acid of (ii); and
(b) determining whether said hybridization has occurred.

9. A process for determining whether or not a test sample originating from or containing human cells has a tumor progression potential, wherein a second sample originating from non-tumor cells from the same individual or a different individual of the same species is also used, which process comprises the following steps:
(a) incubating said samples under stringent hybridization conditions with a nucleic acid probe which is selected from the group consisting of:
(i) a nucleic acid with a sequence of SEQ ID NO:1 or a fragment thereof;
(ii) a nucleic acid with a sequence which is complementary to any nucleic acid of (i);
(iii) a nucleic acid with a sequence which hybridizes under stringent conditions with the nucleic acid of (i); and
(iv) a nucleic acid with a sequence which hybridizes under stringent conditions with the nucleic acid of (ii); and
(b) determining the approximate amount of hybridization of each respective sample with said probe and
(c) comparing the approximate amount of hybridization of the test sample to an approximate amount of hybridization of said second sample to identify whether or not the test sample contains a lower amount of the nucleic acid than does said second sample.

10. A process for determining whether or not a test sample originating from or containing human cells has a tumor progression potential, which process comprises the following steps:
(a) incubating a first compartment of said sample under stringent hybridization conditions with a first nucleic acid probe which is selected from the group consisting of:
(i) a nucleic acid with a sequence of SEQ ID NO:1 or a fragment thereof;
(ii) a nucleic acid with a sequence which is complementary to any nucleic acid of (i);
(iii) a nucleic acid with a sequence which hybridizes under stringent conditions with the nucleic acid of (i); and
(iv) a nucleic acid with a sequence which hybridizes under stringent conditions with the nucleic acid of (ii); and
(b) incubating a second compartment of said sample under stringent hybridization conditions with a second nucleic acid probe being a housekeeping gene or a fragment thereof;
(c) determining the approximate amount of hybridization of said sample with said first and second probe;
(d) identifying whether or not the test sample contains an at least 3-fold amount of nucleic acid hybridizing with the first probe in comparison to the amount of nucleic acid hybridizing with the second probe.

## Patentansprüche

1. Nukleinsäure, die in Säugertumorzellen hochreguliert ist, wobei die Nukleinsäure ausgewählt ist aus der Gruppe, bestehend aus:
(a) SEQ ID Nr.:1;
(b) einer Nukleinsäuresequenz, die frei ist von Sequenzen, die natürlich die 5'-und 3'-Enden der Nukleinsäuresequenz von (a) flankieren, die unter stringenten Bedingungen mit einer Nukleinsäuresonde der komplementären Sequenz von (a) hybridisiert;
(c) einer Nukleinsäuresequenz, die auf Grund der Degeneration des genetischen Codes keine Sequenz von (a) oder (b) ist, jedoch welche für ein Polypeptid codiert, das exakt die gleiche Aminosäuresequenz aufweist wie das Polypeptid, das durch eine Sequenz von (a) oder (b) codiert wird; und
(d) einer Nukleinsäuresequenz, die ein Fragment von mindestens 138 Nukleotiden von einer der Sequenzen (a), (b) oder (c) ist, und für ein Polypeptid codiert, das aus Aminosäuren von SEQ ID Nr.: 2 oder SEQ ID Nr.: 4 besteht,
mit der Maßgabe, dass die Nukleinsäuresequenz nicht identisch ist mit der komplementären Sequenz von SEQ ID Nr.: 12.

2. Nukleinsäure nach Anspruch 1, mit einer Sequenz, die für ein Polypeptid aus Aminosäuren von SEQ ID Nr. 2 oder SEQ ID Nr.: 4 codiert.

3. Nukleinsäure nach Anspruch 1, worin die Nukleinsäure eine Sequenz der Nukleotide 459 bis 848 von SEQ ID Nr.: 1 oder der Nukleotide 1 bis 285 von SEQ ID Nr.: 3 aufweist.

4. Expressionsvektor, umfassend eine Nukleinsäure nach Anspruch 1 bis 3.

5. Wirtszelle, transformiert durch eine Nukleinsäure nach den Ansprüchen 1 bis 3.

6. Polypeptid, das Tumorprogression induziert, worin das Polypeptid codiert wird durch eine Nukleinsäure, ausgewählt aus der Gruppe, bestehend aus:
(a) SEQ ID Nr.: 1;
(b) einer Nukleinsäuresequenz, die unter stringenten Bedingungen mit einer Nukleinsäuresonde der komplementären Sequenz von (a) hybridisiert; und
(c) einer Nukleinsäure, die ein Fragment aus beliebigen mindestens 138 Nukleotiden der Sequenzen von (a) oder (b) ist, und für ein Polypeptid codiert, das aus Aminosäuren der SEQ ID Nr.: 2 oder SEQ ID Nr.: 4 besteht.

7. Polypeptid nach Anspruch 6, worin die Nukleinsäuresequenz von (b) in 6,0 X SSC bei 45 °C bis 55 °C hybridisiert, gefolgt von einem Waschen mit von etwa 2,0 X SSC bis etwa 0,2 X SSC bei einer Temperatur von etwa 50 °C bis etwa 65 °C.

8. Verfahren zum Nachweisen des Vorliegens oder der Abwesenheit mindestens einer spezifischen Nukleinsäure oder eines spezifischen Gemischs von Nukleinsäuren oder zum Unterscheiden zwischen zwei verschiedenen Nukleinsäuren in einer Probe, worin von der Probe vermutet wird, dass sie die Nukleinsäure oder Nukleinsäuren enthält, wobei das Verfahren die folgenden aufeinanderfolgenden Schritte umfasst:
(a) Inkubieren der Probe unter stringenten Hybridisierungsbedingungen mit einer Nukleinsäuresonde, die ausgewählt ist aus der Gruppe, bestehend aus:
(i) einer Nukleinsäure mit einer Sequenz, die genommen ist von der Gruppe, bestehend aus SEQ ID Nr.:1 oder einem Fragment davon;
(ii) einer Nukleinsäure mit einer Sequenz, die komplementär zu einer Nukleinsäure aus (i) ist;
(iii) einer Nukleinsäure mit einer Sequenz, die unter stringenten Bedingungen mit der Nukleinsäure aus (i) hybridisiert; und
(iv) einer Nukleinsäure mit einer Sequenz, die unter stringenten Bedingungen mit der Nukleinsäure aus (ii) hybridisiert; und
(b) Bestimmen ob die Hybridisierung eingetreten ist.

9. Verfahren zum Bestimmen ob eine Testprobe, die von humanen Zellen stammt oder humane Zellen enthält, ein Tumorprogressionspotential aufweist oder ob nicht, worin eine zweite Probe, die von Nicht-Tumorzellen des gleichen Individuums oder eines verschiedenen Individuums der gleiche Spezies stammt, ebenfalls verwendet wird, wobei das Verfahren die folgenden Schritte umfasst:
(a) Inkubieren der Proben unter stringenten Hybridisierungsbedingungen mit einer Nukleinsäuresonde, die ausgewählt ist aus der Gruppe, bestehend aus:
(i) einer Nukleinsäure mit einer Sequenz aus SEQ ID Nr.:1 oder einem Fragment davon;
(ii) einer Nukleinsäure mit einer Sequenz, die komplementär zu einer Nukleinsäure aus (i) ist;
(iii) einer Nukleinsäure mit einer Sequenz, die unter stringenten Bedingungen mit der Nukleinsäure aus (i) hybridisiert; und
(iv) einer Nukleinsäure mit einer Sequenz, die unter stringenten Bedingungen mit der Nukleinsäure aus (ii) hybridisiert; und
(b) Bestimmen des ungefähren Hybrisierungsausmaßes von jeder entsprechenden Probe mit der Sonde und
(c) Vergleichen des ungefähren Hybridisierungsausmaßes der Testprobe mit einem ungefähren Hybridisierungsausmaß der zweiten Probe zum Identifizieren, ob die Testprobe eine geringere Menge der Nukleinsäure als die zweite Probe enthält oder ob nicht.

10. Verfahren zum Bestimmen, ob eine Testprobe, die von humanen Zellen stammt oder humane Zellen enthält, ein Tumorprogressionspotential aufweist oder ob nicht, wobei das Verfahren die folgenden Schritte umfasst:
(a) Inkubieren eines ersten Kompartiments der Probe unter stringenten Hybridisierungsbedingungen mit einer ersten Nukleinsäuresonde, die ausgewählt ist aus der Gruppe, bestehend aus:
(i) einer Nukleinsäure mit einer Sequenz aus SEQ ID Nr.:1 oder einem Fragment davon;
(ii) einer Nukleinsäure mit einer Sequenz, die komplementär zu einer Nukleinsäure aus (i) ist;
(iii) einer Nukleinsäure mit einer Sequenz, die unter stringenten Bedingungen mit der Nukleinsäure aus (i) hybridisiert; und
(iv) einer Nukleinsäure mit einer Sequenz, die unter stringenten Bedingungen mit der Nukleinsäure aus (ii) hybridisiert; und
(b) Inkubieren eines zweiten Kompartiments der Probe unter stringenten Hybridisierungsbedingungen mit einer zweiten Nukleinsäuresonde, die ein Housekeeping-Gen oder ein Fragment davon ist;
(c) Bestimmen des ungefähren Hybrisierungsausmaßes der Probe mit der ersten und zweiten Sonde;
(d) Identifizieren, ob die Testprobe eine mindestens 3-fache Menge Nukleinsäure aufweist oder ob nicht, die mit der ersten Sonde hybridisiert, im Vergleich mit der Menge Nukleinsäure, die mit der zweiten Sonde hybridisiert.

## Revendications

1. Acide nucléique qui est positivement régulé dans les cellules tumorales mammaires, ledit acide nucléique étant choisi dans le groupe consistant en :
(a) SEQ ID NO : 1;
(b) une séquence d'acide nucléique dépourvue des séquences qui flanquent naturellement les extrémités 5' et 3' de la séquence d'acide nucléique de (a) qui s'hybride dans des conditions stringentes avec une sonde d'acide nucléique de la séquence complémentaire de (a) ;
(c) une séquence d'acide nucléique qui, en raison de la dégénérescence du code génétique, n'est pas une séquence de (a) ou (b) mais qui code pour un polypeptide ayant exactement la même séquence d'acides aminés qu'un polypeptide codé par une séquence de (a) ou (b) ; et
(d) une séquence d'acide nucléique qui est un fragment d'au moins 138 nucléotides de l'une quelconque des séquences de (a), (b) ou (c) et qui code pour un polypeptide consistant en acides aminés de SEQ ID. NO : 2 ou de SEQ ID NO : 4,
à condition que ladite séquence d'acide nucléique ne soit pas identique à la séquence complémentaire de SEQ ID NO : 12.

2. Acide nucléique selon la revendication 1, ayant une séquence qui code pour un polypeptide d'acides aminés de SEQ ID NO : 2 ou de SEQ ID NO : 4.

3. Acide nucléique selon la revendication 1, dans lequel l'acide nucléique a une séquence de nucléotides 459 à 848 de SEQ ID NO : 1 ou de nucléotides 1 à 285 de SEQ ID NO : 3.

4. Vecteur d'expression comprenant un acide nucléique selon les revendications 1 à 3.

5. Cellule hôte transformée par un acide nucléique selon les revendications 1 à 3.

6. Polypeptide qui induit la progression d'une tumeur, dans lequel ledit polypeptide est codé par un acide nucléique choisi dans le groupe consistant en :
(a) SEQ ID NO : 1;
(b) une séquence d'acide nucléique qui s'hybride dans des conditions stringentes avec une sonde d'acide nucléique de la séquence complémentaire de (a) ; et
(c) un acide nucléique qui est un fragment d'au moins 138 nucléotides des séquences de (a) ou (b) et qui code pour un polypeptide consistant en acides aminés de SEQ ID NO : 2 ou de SEQ ID NO : 4.

7. Polypeptide selon la revendication 6, dans lequel la séquence d'acide nucléique de (b) s'hybride dans 6,0 x SSC entre 45 °C et 55 °C, suivi par un lavage avec entre environ 2,0 x SSC et environ 0,2 x SSC à une température entre environ 50 °C et environ 65 °C.

8. Procédé permettant de détecter la présence ou l'absence d'au moins un acide nucléique spécifique ou d'un mélange d'acides nucléiques, ou de faire la distinction entre deux acides nucléiques différents dans un échantillon, dans lequel ledit échantillon est soupçonné de contenir ledit ou lesdits acides nucléiques, ce procédé comprenant les étapes suivantes dans l'ordre :
(a) incuber ledit échantillon dans des conditions d'hybridation stringentes avec une sonde d'acide nucléique qui est choisie dans le groupe consistant en :
(i) un acide nucléique dont la séquence est prise dans le groupe consistant en SEQ ID NO : 1 ou un fragment de celle-ci ;
(ii) un acide nucléique avec une séquence qui est complémentaire de n'importe quel acide nucléique de (i) ;
(iii) un acide nucléique avec une séquence qui s'hybride dans des conditions stringentes avec l'acide nucléique de (i) ; et
(iv) un acide nucléique avec une séquence qui s'hybride dans des conditions stringentes avec l'acide nucléique de (ii) ; et
(b) déterminer si ladite hybridation a eu lieu.

9. Procédé permettant de déterminer si un échantillon de test provenant de cellules humaines ou contenant des cellules humaines possède ou non un potentiel de progression de la tumeur, dans lequel un second échantillon provenant de cellules non tumorales du même individu ou d'un individu différent de la même espèce est également utilisé, ce procédé comprenant les étapes suivantes :
(a) incuber lesdits échantillons dans des conditions d'hybridation stringentes avec une sonde d'acide nucléique qui est choisie dans le groupe consistant en :
(i) un acide nucléique avec une séquence de SEQ ID NO : 1 ou un fragment de celle-ci ;
(ii) un acide nucléique avec une séquence qui est complémentaire de n'importe quel acide nucléique de (i) ;
(iii) un acide nucléique avec une séquence qui s'hybride dans des conditions stringentes avec l'acide nucléique de (i) ; et
(iv) un acide nucléique avec une séquence qui s'hybride dans des conditions stringentes avec l'acide nucléique de (ii) ; et
(b) déterminer la quantité approximative d'hybridation de chaque échantillon respectif avec ladite sonde et
(c) comparer la quantité approximative d'hybridation de l'échantillon de test à une quantité approximative d'hybridation dudit second échantillon pour identifier si l'échantillon de test contient ou non une quantité plus faible de l'acide nucléique que ne le fait ledit second échantillon.

10. Procédé permettant de déterminer si un échantillon de test provenant de cellules humaines ou contenant des cellules humaines possède ou non un potentiel de progression de la tumeur, ce procédé comprenant les étapes suivantes :
(a) incuber un premier compartiment dudit échantillon dans des conditions d'hybridation stringentes avec une première sonde d'acide nucléique qui est choisie dans le groupe consistant en :
(i) un acide nucléique avec une séquence de SEQ ID NO : 1 ou un fragment de celle-ci ;
(ii) un acide nucléique avec une séquence qui est complémentaire de n'importe quel acide nucléique de (i) ;
(iii) un acide nucléique avec une séquence qui s'hybride dans des conditions stringentes avec l'acide nucléique de (i) ; et
(iv) un acide nucléique avec une séquence qui s'hybride dans des conditions stringentes avec l'acide nucléique de (ii) ;
(b) incuber un second compartiment dudit échantillon dans des conditions d'hybridation stringentes avec une seconde sonde d'acide nucléique qui est un gène domestique ou un fragment de celui-ci ;
(c) déterminer la quantité approximative d'hybridation dudit échantillon avec lesdites première et seconde sondes ;
(d) identifier si l'échantillon de test contient ou non une quantité au moins 3 fois plus grande d'acide nucléique s'hybridant avec la première sonde par comparaison avec la quantité d'acide nucléique s'hybridant avec la seconde sonde.


### SEQUENCE LISTING

<110> F. Hoffmann-La Roche AG
<120> A nucleic acid which is upregulated in humane tumor cells, a protein encoded thereby and a process for tumor diagnosis
<130> Case 20678
<140>
   <141>
<150> EP00110953.7
   <151> 2000-05-26
<150> EP00115369.1
   <151> 2000-07-15
<160> 12
<170> PatentIn Ver. 2.1
<210> 1
   <211> 2342
   <212> DNA
   <213> Homo sapiens
<220>
   <221> CDS
   <222> (459)..(848)
<400> 1
   aacctccacc acaacgctca cccttacaga cacactattg caggtctccg agggcctttg 60
   ggaggccctg cttcctgcga gctgtcccgg caggacagag actcttcccg ccgcggccct 120
   gccattccag gctgaggctg tgagcagcac catgacaagc tccggccgca gtggctctca 180
   acagtgtggg tctctgacca cccgacgagc tggaagtgca gaccgctgac ctcccttgag 240
   aacctactgg gttcttgcag taggctcctc agcggtgtct aaacacgcca ctcaggtgat 300
   tctatgcacc atcacattgg aaactttttt cattgactgt tacttaatga gaagacttcc 360
   ctccgggatg gttctgaagc ttccttcata ggagcaagcc tttggcggag agcactgagc 420
   agacgtgcag catctttgct ggcttctacc gaaacacc atg gat cca gac gtg gtt 476
                                             Met Asp Pro Asp Val Val
                                              1               5
   ttg tgg tcc tgc acg tgg aag cca gcc ctg cgt ggg gtg agc ctg gga   524
   Leu Trp Ser Cys Thr Trp Lys Pro Ala Leu Arg Gly Val Ser Leu Gly
               10                  15                  20
   ctg tgg gca gag aac ctc aag cac cgg gcc ggc acc caa gtg cag aga    572
   Leu Trp Ala Glu Asn Leu Lys His Arg Ala Gly Thr Gln Val Gln Arg
           25                  30                  35
   ctg cat cgt ccc agc agg agg cgc tgc ttc cag gct ccc tgg acg gac    620
   Leu His Arg Pro Ser Arg Arg Arg Cys Phe Gln Ala Pro Trp Thr Asp
       40                  45                  50
   tcc ggg agg gcg gcc ttt ccc ccc agc ccc aga ggt ggg cct gcc ctt    668
   Ser Gly Arg Ala Ala Phe Pro Pro Ser Pro Arg Gly Gly Pro Ala Leu
    55                  60                  65                  70
   ttc cga gca tgg gac aca gcc cag gaa aac gca tgg ctt gtc ctc cag    716
   Phe Arg Ala Trp Asp Thr Ala Gln Glu Asn Ala Trp Leu Val Leu Gln
                    75                  80                  85
   aca cag gtg cta aca ggg ccg tca gac aag ggc cag gga ctc agg ctt    764
   Thr Gln Val Leu Thr Gly Pro Ser Asp Lys Gly Gln Gly Leu Arg Leu
                90                  95                 100
   tta gga att tca gct cca gag cca cca tgc agt ggg acc agg ggt ctg    812
   Leu Gly Ile Ser Ala Pro Glu Pro Pro Cys Ser Gly Thr Arg Gly Leu
           105                 110                 115
   cgt gga cag gaa gca agc tgt gta gac ggg ggt cca tgaagtagag         858
   Arg Gly Gln Glu Ala Ser Cys Val Asp Gly Gly Pro
       120                 125                 130
   acagggtttt ggggaaggtt ggggcagggc aaggaggaaa agccacattt acagcaattt 918
   ctgaagtctt ttcatttttt ccccctgaat cacgtccata ataggatttg aatttaataa 978
   actgctgaag gttcctggcc ctgagtccca gtgtcctccc agcccccgcc cagctgtggg 1038
   tgtgcatggg gagcggtacg agggagggta aaatgggccc ctgggacgcc gcgtgcagag 1098
   cagagatgaa tggcccgaaa ccctcgcgct gctctgcgcc cttcgtcatc cagtcggggt 1158
   ggttagggac tgtcagagaa aaataattta gcggccatgg ctctaactga tgtgctgcat 1218
   tctggggtca aatgactttt acaaagtagt agtgctgcct ggtttctcta tcgtgagagc 1278
   tcagggctga taacatgaaa gaaaaaggca ctgcagccag aattcactga cattcttcac 1338
   atttcacatg agtgggacgc aggagggggg ctggggaggg tggagggatg tttcctgctt 1398
   aacagattca acagaagagt ggcaggctca gctgggtgag caaggtatcc cagcgacggg 1458
   ggacacgccc cagaccatgg gtggtggggc ttctcagagg aggtggcagg agacccgagc 1518
   ctgccaaggt tgcacctaag gtcacgggca gcattaggag ggctctctcc cagtctcccc 1578
   acccccccgt cccccctccc ccaggctgca ggggtgaagt ggcttccagg acggtcactg 1638
   gcaagtttaa gctacagaga gtgtagaaac agggtgaaaa aggaagagag aggggagtaa 1698
   ataagaagga ggtgtaagaa aagaccaagc caggccccag cgcccttgtg aggaagtgcc 1758
   cagggactta tgtggaagcc gtccttgctg tctgccacct tgtttttact tacattgtgt 1818
   ttttatttga gggcgagttt ggacggcaag actgatggag attgtggtct aaatgcctct 1878
   aacccactcc ttaaaatgac caccggatgt tccacaagta cttgaaaatg aatgaatggc 1938
   ttcccgagag gcagaaggca ggggtgtgcc ctaccccacg ccggccaaga gttcaacaag 1998
   cattggttga caagtgaata gtgagcactt gaacccagtc acaattcaag atgagggctc 2058
   tgccatgacg catgtggtct gtgtcaccct gcagtctccc tgagcagtgt ctgaggttcg 2118
   agtgggaccc tacattcgtg aagagattta tcatctcccc aggaaaaata acagattctg 2178
   tcctaggtgt tgtgatgtaa caatggtagc gatcacagcc ataacttaca attattgcat 2238
   acttacgacg agtcccgcac tgggctaagt gctttttaac tatgtgaaat gtttctttcc 2298
   ttgattgatg ccaaaatgaa taaagataat tttctgtatc tgct                 2342
<210> 2
   <211> 130
   <212> PRT
   <213> Homo sapiens
<400> 2
   Met Asp Pro Asp Val Val Leu Trp Ser Cys Thr Trp Lys Pro Ala Leu
   1               5                   10                  15
   Arg Gly Val Ser Leu Gly Leu Trp Ala Glu Asn Leu Lys His Arg Ala
               20                  25                  30
   Gly Thr Gln Val Gln Arg Leu His Arg Pro Ser Arg Arg Arg Cys Phe
           35                  40                  45
   Gln Ala Pro Trp Thr Asp Ser Gly Arg Ala Ala Phe Pro Pro Ser Pro
       50                  55                  60
   Arg Gly Gly Pro Ala Leu Phe Arg Ala Trp Asp Thr Ala Gln Glu Asn
   65                  70                  75                  80
   Ala Trp Leu Val Leu Gln Thr Gln Val Leu Thr Gly Pro Ser Asp Lys
                   85                  90                  95
   Gly Gln Gly Leu Arg Leu Leu Gly Ile Ser Ala Pro Glu Pro Pro Cys
               100                 105                 110
   Ser Gly Thr Arg Gly Leu Arg Gly Gln Glu Ala Ser Cys Val Asp Gly
           115                 120                 125
   Gly Pro
       130
<210> 3
   <211> 285
   <212> DNA
   <213> Homo sapiens
<220>
   <221> CDS
   <222> (1)..(285)
<400> 3
   atg gat cca gac gtg gtt ttg tgg tcc tgc acg tgg aag cca gcc ctg     48
   Met Asp Pro Asp Val Val Leu Trp Ser Cys Thr Trp Lys Pro Ala Leu
     1               5                  10                  15
   cgt ggg gtg agc ctg gga ctg tgg gca gag aac ctc aag cac cgg gcc     96
   Arg Gly Val Ser Leu Gly Leu Trp Ala Glu Asn Leu Lys His Arg Ala
                20                  25                  30
   ggc acc caa gtg cag aga ctg cat cgt ccc aac agg agg cgc tgc ttc    144
   Gly Thr Gln Val Gln Arg Leu His Arg Pro Asn Arg Arg Arg Cys Phe
            35                  40                  45
   cag gct ccc tgg acg gac tcc ggg agg gcg gcc ttt ccc ccc agc ccc    192
   Gln Ala Pro Trp Thr Asp Ser Gly Arg Ala Ala Phe Pro Pro Ser Pro
        50                  55                  60
   aga ggt ggg cct gcc ctt ttc cga gcg tgg gac aca gcc cag gaa aac    240
   Arg Gly Gly Pro Ala Leu Phe Arg Ala Trp Asp Thr Ala Gln Glu Asn
    65                  70                  75                  80
   gca tgg ctt gtc ctc cag aca cag ggc gag ttt gga cgg caa gac        285
   Ala Trp Leu Val Leu Gln Thr Gln Gly Glu Phe Gly Arg Gln Asp
                    85                  90                  95
<210> 4
   <211> 95
   <212> PRT
   <213> Homo sapiens
<400> 4
   Met Asp Pro Asp Val Val Leu Trp Ser Cys Thr Trp Lys Pro Ala Leu
     1               5                  10                  15
   Arg Gly Val Ser Leu Gly Leu Trp Ala Glu Asn Leu Lys His Arg Ala
                20                  25                  30
   Gly Thr Gln Val Gln Arg Leu His Arg Pro Asn Arg Arg Arg Cys Phe
            35                  40                  45
   Gln Ala Pro Trp Thr Asp Ser Gly Arg Ala Ala Phe Pro Pro Ser Pro
        50                  55                  60
   Arg Gly Gly Pro Ala Leu Phe Arg Ala Trp Asp Thr Ala Gln Glu Asn
    65                  70                  75                  80
   Ala Trp Leu Val Leu Gln Thr Gln Gly Glu Phe Gly Arg Gln Asp
                    85                  90                  95
<210> 5
   <211> 19
   <212> DNA
   <213> Artificial Sequence
<220>
   <223> Description of Artificial Sequence:primer GSP1
<400> 5
   ttatctttat tcattttgg 19
<210> 6
   <211> 23
   <212> DNA
   <213> Artificial Sequence
<220>
   <223> Description of Artificial Sequence:primer GSP2
<400> 6
   tgcgggactc gtcgtaagta tgc 23
<210> 7
   <211> 20
   <212> DNA
   <213> Artificial Sequence
<220>
   <223> Description of Artificial Sequence:primer AUAP
<400> 7
   ggccacgcgt cgactagtac 20
<210> 8
   <211> 19
   <212> DNA
   <213> Artificial Sequence
<220>
   <223> Description of Artificial Sequence:primer RTR-5
<400> 8
   ccattcattc attttcaag 19
<210> 9
   <211> 17
   <212> DNA
   <213> Artificial Sequence
<220>
   <223> Description of Artificial Sequence:primer RTF-6
<400> 9
   aaaacgcatg gcttgtc 17
<210> 10
   <211> 25
   <212> DNA
   <213> Artificial Sequence
<220>
   <223> Description of Artificial Sequence: -actin reverse primer
<400> 10
   agggtacatg gtggtgccgc cagac 25
<210> 11
   <211> 25
   <212> DNA
   <213> Artificial Sequence
<220>
   <223> Description of Artificial Sequence: -actin forward primer
<400> 11
   ccaaggccaa ccgcgagaag atgac 25
<210> 12
   <211> 127
   <212> DNA
   <213> Homo sapiens
<220>
   <223> fragment of sequence AQ548392, nuclotide 1 correspond to nucleotide 304 and nucleotide 127 correspond to nucleotide 430 of the complete sequence
<300>
   <308> AQ548392
<400> 12
   tggacccccg tctacacagc ttgcttcctg tccactcaga cccctggtcc cactgcatgg 60
   tggctctgga gctgaaattc ctaaaagcct gagtccctgg cccttgtctg acggccctgt 120
   tagcacc                                                           127

### SEQUENCE LISTING

<110> F. Hoffmann-La Roche AG
<120> A nucleic acid which is upregulated in human tumor cells, a protein encoded thereby and a process for tumor diagnosis
<130> Case 20678
<140>
   <141>
<150> EP00110953.7
   <151> 2000-05-26
<150> EP00115369.1
   <151> 2000-07-15
<160> 12
<170> PatentIn Ver. 2.1
<210> 1
   <211> 2342
   <212> DNA
   <213> Homo sapiens
<220>
   <221> CDS
   <222> (459)..(848)
<400> 1
   aacctccacc acaacgctca cccttacaga cacactattg caggtctccg agggcctttg 60
   ggaggccctg cttcctgcga gctgtcccgg caggacagag actcttcccg ccgcggccct 120
   gccattccag gctgaggctg tgagcagcac catgacaagc tccggccgca gtggctctca 180
   acagtgtggg tctctgacca cccgacgagc tggaagtgca gaccgctgac ctcccttgag 240
   aacctactgg gttcttgcag taggctcctc agcggtgtct aaacacgcca ctcaggtgat 300
   tctatgcacc atcacattgg aaactttttt cattgactgt tacttaatga gaagacttcc 360
   ctccgggatg gttctgaagc ttccttcata ggagcaagcc tttggcggag agcactgagc 420
   agacgtgcag catctttgct ggcttctacc gaaacacc atg gat cca gac gtg gtt 476
                                             Met Asp Pro Asp Val Val
                                             1               5
   ttg tgg tcc tgc acg tgg aag cca gcc ctg cgt ggg gtg agc ctg gga   524
   Leu Trp Ser Cys Thr Trp Lys Pro Ala Leu Arg Gly Val Ser Leu Gly
               10                  15                  20
   ctg tgg gca gag aac ctc aag cac cgg gcc ggc acc caa gtg cag aga   572
   Leu Trp Ala Glu Asn Leu Lys His Arg Ala Gly Thr Gln Val Gln Arg
            25                  30                  35
   ctg cat cgt ccc agc agg agg cgc tgc ttc cag gct ccc tgg acg gac   620
   Leu His Arg Pro Ser Arg Arg Arg Cys Phe Gln Ala Pro Trp Thr Asp
        40                  45                  50
   tcc ggg agg gcg gcc ttt ccc ccc agc ccc aga ggt ggg cct gcc ctt   668
   Ser Gly Arg Ala Ala Phe Pro Pro Ser Pro Arg Gly Gly Pro Ala Leu
    55                  60                  65                  70
   ttc cga gca tgg gac aca gcc cag gaa aac gca tgg ctt gtc ctc cag   716
   Phe Arg Ala Trp Asp Thr Ala Gln Glu Asn Ala Trp Leu Val Leu Gln
                    75                  80                  85
   aca cag gtg cta aca ggg ccg tca gac aag ggc cag gga ctc agg ctt   764
   Thr Gln Val Leu Thr Gly Pro Ser Asp Lys Gly Gln Gly Leu Arg Leu
                90                  95                 100
   tta gga att tca gct cca gag cca cca tgc agt ggg acc agg ggt ctg   812
   Leu Gly Ile Ser Ala Pro Glu Pro Pro Cys Ser Gly Thr Arg Gly Leu
           105                 110                 115
   cgt gga cag gaa gca agc tgt gta gac ggg ggt cca tgaagtagag        858
   Arg Gly Gln Glu Ala Ser Cys Val Asp Gly Gly Pro
       120                 125                 130
   acagggtttt ggggaaggtt ggggcagggc aaggaggaaa agccacattt acagcaattt 918
   ctgaagtctt ttcatttttt ccccctgaat cacgtccata ataggatttg aatttaataa 978
   actgctgaag gttcctggcc ctgagtccca gtgtcctccc agcccccgcc cagctgtggg 1038
   tgtgcatggg gagcggtacg agggagggta aaatgggccc ctgggacgcc gcgtgcagag 1098
   cagagatgaa tggcccgaaa ccctcgcgct gctctgcgcc cttcgtcatc cagtcggggt 1158
   ggttagggac tgtcagagaa aaataattta gcggccatgg ctctaactga tgtgctgcat 1218
   tctggggtca aatgactttt acaaagtagt agtgctgcct ggtttctcta tcgtgagagc 1278
   tcagggctga taacatgaaa gaaaaaggca ctgcagccag aattcactga cattcttcac 1338
   atttcacatg agtgggacgc aggagggggg ctggggaggg tggagggatg tttcctgctt 1398
   aacagattca acagaagagt ggcaggctca gctgggtgag caaggtatcc cagcgacggg 1458
   ggacacgccc cagaccatgg gtggtggggc ttctcagagg aggtggcagg agacccgagc 1518
   ctgccaaggt tgcacctaag gtcacgggca gcattaggag ggctctctcc cagtctcccc 1578
   acccccccgt cccccctccc ccaggctgca ggggtgaagt ggcttccagg acggtcactg 1638
   gcaagtttaa gctacagaga gtgtagaaac agggtgaaaa aggaagagag aggggagtaa 1698
   ataagaagga ggtgtaagaa aagaccaagc caggccccag cgcccttgtg aggaagtgcc 1758
   cagggactta tgtggaagcc gtccttgctg tctgccacct tgtttttact tacattgtgt 1818
   ttttatttga gggcgagttt ggacggcaag actgatggag attgtggtct aaatgcctct 1878
   aacccactcc ttaaaatgac caccggatgt tccacaagta cttgaaaatg aatgaatggc 1938
   ttcccgagag gcagaaggca ggggtgtgcc ctaccccacg ccggccaaga gttcaacaag 1998
   cattggttga caagtgaata gtgagcactt gaacccagtc acaattcaag atgagggctc 2058
   tgccatgacg catgtggtct gtgtcaccct gcagtctccc tgagcagtgt ctgaggttcg 2118
   agtgggaccc tacattcgtg aagagattta tcatctcccc aggaaaaata acagattctg 2178
   tcctaggtgt tgtgatgtaa caatggtagc gatcacagcc ataacttaca attattgcat 2238
   acttacgacg agtcccgcac tgggctaagt gctttttaac tatgtgaaat gtttctttcc 2298
   ttgattgatg ccaaaatgaa taaagataat tttctgtatc tgct                  2342
<210> 2
   <211> 130
   <212> PRT
   <213> Homo sapiens
<400> 2
   Met Asp Pro Asp Val Val Leu Trp Ser Cys Thr Trp Lys Pro Ala Leu
   1               5                   10                  15
   Arg Gly Val Ser Leu Gly Leu Trp Ala Glu Asn Leu Lys His Arg Ala
               20                  25                  30
   Gly Thr Gln Val Gln Arg Leu His Arg Pro Ser Arg Arg Arg Cys Phe
           35                  40                  45
   Gln Ala Pro Trp Thr Asp Ser Gly Arg Ala Ala Phe Pro Pro Ser Pro
       50                  55                  60
   Arg Gly Gly Pro Ala Leu Phe Arg Ala Trp Asp Thr Ala Gln Glu Asn
   65                  70                  75                  80
   Ala Trp Leu Val Leu Gln Thr Gln Val Leu Thr Gly Pro Ser Asp Lys
                   85                  90                  95
   Gly Gln Gly Leu Arg Leu Leu Gly Ile Ser Ala Pro Glu Pro Pro Cys
               100                 105                 110
   Ser Gly Thr Arg Gly Leu Arg Gly Gln Glu Ala Ser Cys Val Asp Gly
           115                 120                 125
   Gly Pro
       130
<210> 3
   <211> 285
   <212> DNA
   <213> Homo sapiens
<220>
   <221> CDS
   <222> (1)..(285)
<400> 3
   atg gat cca gac gtg gtt ttg tgg tcc tgc acg tgg aag cca gcc ctg    48
   Met Asp Pro Asp Val Val Leu Trp Ser Cys Thr Trp Lys Pro Ala Leu
    1               5                  10                  15
   cgt ggg gtg agc ctg gga ctg tgg gca gag aac ctc aag cac cgg gcc    96
   Arg Gly Val Ser Leu Gly Leu Trp Ala Glu Asn Leu Lys His Arg Ala
               20                  25                  30
   ggc acc caa gtg cag aga ctg cat cgt ccc aac agg agg cgc tgc ttc   144
   Gly Thr Gln Val Gln Arg Leu His Arg Pro Asn Arg Arg Arg Cys Phe
           35                  40                  45
   cag gct ccc tgg acg gac tcc ggg agg gcg gcc ttt ccc ccc agc ccc   192
   Gln Ala Pro Trp Thr Asp Ser Gly Arg Ala Ala Phe Pro Pro Ser Pro
       50                  55                  60
   aga ggt ggg cct gcc ctt ttc cga gcg tgg gac aca gcc cag gaa aac   240
   Arg Gly Gly Pro Ala Leu Phe Arg Ala Trp Asp Thr Ala Gln Glu Asn
   65                  70                  75                  80
   gca tgg ctt gtc ctc cag aca cag ggc gag ttt gga cgg caa gac       285
   Ala Trp Leu Val Leu Gln Thr Gln Gly Glu Phe Gly Arg Gln Asp
                   85                  90                  95
<210> 4
   <211> 95
   <212> PRT
   <213> Homo sapiens
<400> 4
   Met Asp Pro Asp Val Val Leu Trp Ser Cys Thr Trp Lys Pro Ala Leu
    1               5                  10                  15
   Arg Gly Val Ser Leu Gly Leu Trp Ala Glu Asn Leu Lys His Arg Ala
               20                  25                  30
   Gly Thr Gln Val Gln Arg Leu His Arg Pro Asn Arg Arg Arg Cys Phe
           35                  40                  45
   Gln Ala Pro Trp Thr Asp Ser Gly Arg Ala Ala Phe Pro Pro Ser Pro
       50                  55                  60
   Arg Gly Gly Pro Ala Leu Phe Arg Ala Trp Asp Thr Ala Gln Glu Asn
   65                  70                  75                  80
   Ala Trp Leu Val Leu Gln Thr Gln Gly Glu Phe Gly Arg Gln Asp
                   85                  90                  95
<210> 5
   <211> 19
   <212> DNA
   <213> Artificial Sequence
<220>
   <223> Description of Artificial Sequence:primer GSP1
<400> 5
   ttatctttat tcattttgg 19
<210> 6
   <211> 23
   <212> DNA
   <213> Artificial Sequence
<220>
   <223> Description of Artificial Sequence:primer GSP2
<400> 6
   tgcgggactc gtcgtaagta tgc 23
<210> 7
   <211> 20
   <212> DNA
   <213> Artificial Sequence
<220>
   <223> Description of Artificial Sequence:primer AUAP
<400> 7
   ggccacgcgt cgactagtac 20
<210> 8
   <211> 19
   <212> DNA
   <213> Artificial Sequence
<220>
   <223> Description of Artificial Sequence:primer RTR-5
<400> 8
   ccattcattc attttcaag 19
<210> 9
   <211> 17
   <212> DNA
   <213> Artificial Sequence
<220>
   <223> Description of Artificial Sequence:primer RTF-6
<400> 9
   aaaacgcatg gcttgtc 17
<210> 10
   <211> 25
   <212> DNA
   <213> Artificial Sequence
<220>
   <223> Description of Artificial Sequence:-actin reverse primer
<400> 10
   agggtacatg gtggtgccgc cagac 25
<210> 11
   <211> 25
   <212> DNA
   <213> Artificial Sequence
<220>
   <223> Description of Artificial Sequence: -actin forward primer
<400> 11
   ccaaggccaa ccgcgagaag atgac 25
<210> 12
   <211> 127
   <212> DNA
   <213> Homo sapiens
<220>
   <223> fragment of sequence AQ548392, nuclotide 1 correspond to nucleotide 304 and nucleotide 127 correspond to nucleotide 430 of the complete sequence
<300>
   <308> AQ548392
<400> 12
   tggacccccg tctacacagc ttgcttcctg tccactcaga cccctggtcc cactgcatgg 60
   tggctctgga gctgaaattc ctaaaagcct gagtccctgg cccttgtctg acggccctgt 120
   tagcacc                                                           127